# EUROPEAN PATENT APPLICATION

(11) **EP 4 545 088 A1**
(43) Date of publication of application: **30.04.2025**
(21) Application number: 23827566.3
(22) Date of filing: 23.06.2023
(51) Int. Cl.: A61K 38/17, A61K 38/08, A61K 47/55, A61P 39/06, A61P 17/00, C07K 7/06, A23L 33/18

(54) **COMPOSITION COMPRISING CPNE7 PROTEIN OR CPNE7 PROTEIN-DERIVED PEPTIDE FOR REMOVING REACTIVE OXYGEN SPECIES IN CELLS**

(30) Priority: 24.06.2022 KR 20220077822; 17.04.2023 KR 20230050379
(71) Applicant: Hysensbio Co., Ltd, Gwacheon-si Gyeonggi-do 13814 (KR)
(72) Inventor: PARK, Joo Hwang, Incheon 21986 (KR); PARK, Yeoung Hyun, Seoul 04378 (KR)
(74) Representative: Mewburn Ellis LLP
(86) International application number: PCT/KR2023/008779
(87) International publication number: WO 2023/249458

(57) **Abstract**

The present invention relates to a composition for scavenging intracellular reactive oxygen species, which includes a CPNE7 protein or a functional peptide (Selcopintide) derived from the CPNE7 protein, a polynucleotide encoding the peptide, an expression vector including the polynucleotide, and a pharmaceutical composition, a quasi-drug composition, and a health functional food composition including the peptide.

## Description

### [Technical Field]

The present invention relates to a composition for scavenging reactive oxygen species, and more particularly, to a composition for scavenging intracellular reactive oxygen species, which includes a CPNE7 protein or a peptide derived from the CPNE7 protein.

### [Background Art]

Advances in medical technology have extended the average lifespan of humans through the prevention and treatment of diseases. With the recent growth of the elderly population, interest in the prevention and treatment of various geriatric diseases has increased. However, the exact mechanism causing aging has not yet been identified, and technology related to the cause and treatment of many geriatric diseases are still in the early stage of development. According to the "2021 Elderly Statistics" of the National Statistics Office (NSO) in Korea, people aged 65 or older account for 16.5% of the total population, and are expected to increase every year. An aging society as defined by the UN is a society in which people aged 65 or older account for more than 7% of the total population. Based on this survey, Korea has already entered an aging society, and the proportion of the elderly population is expected to increase further as the average life expectancy continues to increase.

As the elderly population increases, the development of anti-aging technology that enables the prevention and treatment of geriatric diseases has become increasingly important. To understand aging at the individual level in relation to anti-aging technology, it is necessary to first clarify the aging phenomenon from a cellular-molecular biological perspective. According to the free radical theory (Hartman *et al.,* 1986), which is accepted as the most influential study on the cause of aging, reactive oxygen species (ROS) with strong reactivity cause somatic mutations to damage proteins, which is considered to be a cause of aging. Reactive oxygen species are structurally very unstable and thus can cause strong oxidation when combined with intracellular DNA. Highly toxic oxygen radicals combine with polyunsaturated fatty acids (PUFAs) in the cell membrane to cause changes in the cell membrane, thereby ultimately promoting cell aging. Not only are numerous diseases related to reactive oxygen species, such as tissue damage caused by ischemia/reperfusion, metabolic syndrome, degenerative neurological diseases such as Parkinson's disease and Alzheimer's disease, premature aging, cancer, and cardiovascular diseases, but the reactive oxygen species also accelerate skin aging by changing the form of collagen and elastin, which are mainly present in the dermis of the skin.

Endogenous antioxidant enzymes (SOD, catalase, glutathione peroxidase, etc.) naturally produced in the human body function to neutralize reactive oxygen species by stably changing the structure of the reactive oxygen species. However, this is reported to decrease after the age of 30, and it is known to decrease by approximately 50% at the age of 40 and up to 90% by the age of 60 as compared to the age of 25. Therefore, the production rate of reactive oxygen species increases as aging progresses. Secondary antioxidants, which are non-enzymatic exogenous antioxidant substances (vitamins C, E, A, selenium, etc.), are rapidly absorbed after ingestion, and thus cannot sufficiently compensate for the decrease in endogenous antioxidant enzymes.

It was experimentally confirmed that aging of odontoblasts forming dentin was accelerated in Copine-7 (CPNE7) protein-deficient mice, which results in the formation of pathological dentin. CPNE7 is a protein that induces the regeneration of dentin, which constitutes approximately 70% or more of the tooth structure. Based on the fact that the CPNE7 protein is an essential element for maintaining homeostasis in odontoblasts, the present inventors have found that CPNE7 deficiency would lead to DNA damage by causing excessive formation of reactive oxygen species due to a decrease in endogenous antioxidant enzymes in odontoblasts. Therefore, the present invention has been completed based on these facts.

### [Disclosure]

### [Technical Problem]

Accordingly, it is an object of the present invention to provide a composition for scavenging intracellular reactive oxygen species, which includes a CPNE7 protein or a peptide derived from the CPNE7 protein.

It is another object of the present invention to provide a polynucleotide encoding the peptide.

It is still another object of the present invention to provide an expression vector including the polynucleotide.

It is yet another object of the present invention to provide a pharmaceutical composition for preventing or treating a heart disease caused by oxidative stress in cardiomyocytes or a skin disease caused by oxidative stress in dermal fibroblasts, which includes the peptide.

It is yet another object of the present invention to provide a quasi-drug composition for preventing or ameliorating a heart disease caused by oxidative stress in cardiomyocytes or a skin disease caused by oxidative stress in dermal fibroblasts, which includes the peptide.

It is yet another object of the present invention to provide a health functional food composition for preventing or ameliorating a heart disease caused by oxidative stress in cardiomyocytes or a skin disease caused by oxidative stress in dermal fibroblasts, which includes the peptide.

The objects of the present invention are not limited to those mentioned above, and other objects not mentioned herein will be clearly understood by those skilled in the art to which the present invention pertains from the description below.

### [Technical Solution]

To solve the above technical problems, the present inventors have conducted various studies and developed a composition for scavenging intracellular reactive oxygen species, which includes a CPNE7 protein or a CPNE7 protein-derived peptide that scavenges the intracellular reactive oxygen species.

Also, the present inventors have developed a novel CPNE7 protein-derived peptide consisting of 10 amino acids for the preparation of a formulation that may treat a heart disease caused by oxidative stress in cardiomyocytes or a skin disease caused by oxidative stress in dermal fibroblasts.

According to an aspect of the present invention, there is provided a peptide for treating a heart disease caused by oxidative stress in cardiomyocytes or a skin disease caused by oxidative stress in dermal fibroblasts, which includes an amino acid sequence of the following General Formula 1:

[General Formula 1] K-Y-R1-R2-R3-R4-R5-R6-R7-R8

wherein:
R1 is arginine (R), lysine (K), or glutamine (Q);
R2 is arginine (R) or glutamine (Q);
R3, R4, and R5 are each arginine (R) or lysine (K);
R6 is asparagine (N) or serine (S); and
R7 and R8 are lysine (K) or tyrosine (Y).

The peptide provided in the present invention may increase the expression levels of Dspp, Dmp1 and Nestin genes, which are odontoblast differentiation marker genes, without exhibiting cytotoxicity. In this case, when the peptide is transplanted *in vivo* together with dental pulp tissue cells, the dental pulp tissue cells may exhibit the characteristic of forming dentin/dental pulp tissue-like tissue.

As long as the peptide provided in the present invention can exhibit a therapeutic effect on a heart disease caused by oxidative stress in cardiomyocytes or a skin disease caused by oxidative stress in dermal fibroblasts, a variant peptide having a sequence that differs by one or more amino acid residues from an amino acid sequence constituting the peptide is also included in the category of the peptide provided in the present invention.

In general, amino acid exchanges in proteins and polypeptides that do not alter the overall activity of the molecule are well known in the art. The most commonly occurring exchanges are exchanges between amino acid residues Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, and Asp/Gly. Also, peptides whose structural stability against heat, pH, and the like is increased, or whose ability to scavenge intracellular reactive oxygen species is increased by mutations or modifications in the amino acid sequence may be included.

For example, even when glutamine, which is an acidic amino acid located at position 3 of the peptide of SEQ ID NO: 1 provided in the present invention, is substituted with lysine or arginine, which are basic amino acids, the effect of the peptide provided in the present invention may be exhibited as is; even when arginine, which is a basic amino acid located at position 4 or 5 of the peptide of SEQ ID NO: 1, is substituted with glutamine, which is an acidic amino acid, or lysine, which is a basic amino acid, the effect of the peptide provided in the present invention may be exhibited as is; even when lysine, which is a basic amino acid located at position 6, 7 or 9 of the peptide of SEQ ID NO: 1, is substituted with arginine, which is a basic amino acid, or tyrosine, which is an aromatic amino acid, the effect of the peptide provided in the present invention may be exhibited as is; even when asparagine, an acidic amino acid located at position 8 of the peptide of SEQ ID NO: 1, is substituted with serine, which is a neutral amino acid, the effect of the peptide provided in the present invention may be exhibited as is; and even when tyrosine, which is an aromatic amino acid located at position 10 of the peptide of SEQ ID NO: 1, is substituted with lysine, which is a basic amino acid, the effect of the peptide provided in the present invention may be exhibited as is.

In this way, since the effect of the peptide provided in the present invention may be exhibited as is even when an acidic, basic or aromatic amino acid constituting the peptide of the present invention is substituted with another acidic, basic, neutral, or aromatic amino acid, it is clear that a variant peptide having a sequence that differs by one or more amino acid residues from the amino acid sequence constituting the peptide of the present invention is also included in the category of the peptide provided in the present invention.

Also, since the effect of the peptide provided in the present invention may be exhibited as is even when the peptide of the present invention has a form in which any amino acid is added to the N-terminus or C-terminus thereof, it is included in the category of the peptide provided in the present invention. As an example, the peptide may have a form in which 1 to 300 amino acids are added to the N-terminus or C-terminus of the peptide. As another example, the peptide may have a form in which 1 to 100 amino acids are added to the N-terminus or C-terminus of the peptide. As still another example, the peptide may have a form in which 1 to 24 amino acids are added to the N-terminus or C-terminus of the peptide.

In another aspect, the present invention provides a polynucleotide encoding the peptide.

The polynucleotide may be mutated by substitution, deletion, or insertion of one or more bases, or a combination thereof. When the nucleotide sequence is chemically synthesized, a synthetic method widely known in the art, for example, a method described in Engels and Uhlmann, Angew Chem IntEd Engl., 37:73-127, 1988, may be used. Also, the nucleotide sequence may be synthesized using triester, phosphite, phosphoramidite, and H-phosphate methods, PCR and other autoprimer methods, methods of synthesizing an oligonucleotide on a solid support, and the like. For example, a polynucleotide encoding the peptide of the present invention may include a base sequence of SEQ ID NO: 4.

In still another aspect, the present invention provides an expression vector including the polynucleotide, a transformant including the expression vector, and a method of preparing the peptide using the transformant.

The term "expression vector" of the present invention refers to a recombinant vector capable of expressing a target peptide in a target host cell, and means a genetic construct including essential regulatory elements operably linked to allow a gene insert to be expressed. The expression vector includes expression regulatory elements such as an initiation codon, a stop codon, a promoter, an operator, and the like. In this case, the initiation codon and the stop codon are generally considered to be part of a nucleotide sequence encoding the polypeptide, and must be functional in an individual when a genetic construct is administered, and must be in frame with the coding sequence. The promoter of the vector may be constitutive or inducible.

The term "operably linked" of the present invention means a state in which a nucleic acid expression regulatory sequence and a nucleic acid sequence encoding a target protein or RNA are functionally linked to perform a general function. For example, a promoter and a nucleic acid sequence encoding a protein or RNA are operably linked to affect the expression of the coding sequence. An operably linked expression vector may be produced using a recombinant DNA technique well known in the art, and site-specific DNA cleavage and linkage may be performed using enzymes generally known in the art.

Also, the expression vector may include a signal sequence for releasing the peptide in order to facilitate isolation of the peptide from the cell culture. Specific initiation signals may also be required for efficient translation of the inserted nucleic acid sequence. These signals include an ATG start codon and adjacent sequences. In some cases, an exogenous translational control signal, which may include the ATG start codon, must be provided. These exogenous translational control signals and the initiation codon may be derived from a variety of natural and synthetic sources. Expression efficiency may be increased by the introduction of appropriate transcriptional or translational enhancing factors.

In addition, the expression vector may further include a protein tag that may be optionally removed using an endopeptidase to facilitate detection of the peptide.

The term "tag" of the present invention refers to a molecule that exhibits a quantifiable activity or characteristics, and may be a fluorescent molecule including a chemical fluorescent material such as fluorescein (i.e., fluoracer), a polypeptide fluorescent material such as a fluorescent protein (GFP) or related proteins; or may be an epitope tag such as a Myc tag, a Flag tag, a histidine tag, a leucine tag, an IgG tag a straptavidin tag, or the like. In particular, when the epitope tag is used, a peptide tag preferably consisting of 6 or more amino acid residues, and more preferably consisting of 8 to 50 amino acid residues, may be used.

The term "coronary artery disease (CAD)" of the present invention refers to a disease in which the coronary artery, which is the main blood vessel of the heart, is narrowed or blocked. This can occur when the heart muscle is insufficiently supplied with oxygen and nutrients. This phenomenon is mainly caused by arteriosclerosis. Arteriosclerosis occurs when cholesterol, fat, calcium, and the like are deposited on the blood vessel walls. Here, oxidative stress plays a major role in this process. Oxidative stress is a disease caused by oxidative stress in cardiomyocytes that worsens blood vessel damage and induces inflammation, thereby accelerating arteriosclerosis.

The term "myocardial infarction (MI)" of the present invention may refer to a condition in which the blood supply of the coronary artery is suddenly blocked, causing the heart muscle to stop. Oxidative stress may worsen damage to cardiomyocytes and promote inflammatory reactions, thereby causing a decline in heart function.

The term "heart failure" of the present invention refers to a condition in which the heart cannot supply sufficient blood to the body. In this situation, oxidative stress in cardiomyocytes is one of the main causes of heart failure, which may worsen cell damage and inflammation and lower heart function.

The term "atrial fibrillation" of the present invention may refer to a condition in which an abnormal electrical signal is generated in the atria of the heart, causing the heart rhythm to become fast and irregular, which in turn prevents the heart from efficiently supplying blood to the body. Oxidative stress in cardiomyocytes causes an imbalance in the cells, increasing oxygen-reactive compounds (free radicals), and these oxidative compounds damage cells and biomolecules, thereby worsening the electrical imbalance of the heart and increasing the risk of developing atrial fibrillation.

Oxidative stress in cardiomyocytes may aggravate cardiac damage and inflammation, which may impair the heart's electrical stability and cause heart disease, which makes it possible to treat heart disease by reducing oxidative stress in cardiomyocytes.

The term "photodermatitis" of the present invention may refer to a condition in which ultraviolet rays or a specific substance combines with sunlight to cause an inflammatory reaction in the skin, and one of the main causes of skin damage caused by ultraviolet exposure is oxidative stress. Oxidative stress may affect dermal fibroblasts, which may promote collagen breakdown and inflammatory reactions. This process may change the characteristics of the skin and worsen the symptoms of photodermatitis.

The term "atopic dermatitis" of the present invention is a chronic dermatitis characterized by dry skin, itching, and repeated inflammation, and is known to be associated with genetic factors, environmental factors, immune system abnormalities, and the like. Stress in dermal fibroblasts may be associated with the onset and progression of atopic dermatitis. Oxidative stress generates free radicals that damage cells, which causes inflammatory reactions and a decrease in cell function. Since dermal fibroblasts are located in the dermal layer of the skin and play an important role in maintaining the structure and function of the skin, the protective function of the skin may be weakened when the function of dermal fibroblasts is reduced due to oxidative stress. As a result, the skin may become more vulnerable to external stimuli, and the symptoms of atopic dermatitis may worsen. The oxidative stress generated in this process may promote inflammatory reactions. This inflammatory reaction is one of the main symptoms of atopic dermatitis, and causes dermal fibroblasts to not function properly. In addition, cell damage caused due to oxidative stress may worsen damage to the skin barrier. When the skin barrier is damaged, substances that cause allergic reactions may more easily penetrate the skin, which may further worsen the symptoms of atopic dermatitis.

In the present invention, the expression vector may include a nucleotide sequence encoding a CPNE7 protein or a peptide consisting of amino acids set forth in SEQ ID NO: 1. In this case, the vector used herein is not particularly limited as long as it may produce the peptide, and may preferably be a plasmid DNA, phage DNA, or the like, and more preferably a commercially developed plasmid (pUC18, pBAD, pIDTSAMRT-AMP, etc.), an *E. coli-*derived plasmid (pYG601BR322, pBR325, pUC118, pUC119, etc.), a *Bacillus subtilis-*derived plasmid (pUB110, pTP5, etc.), a yeast-derived plasmid (YEp13, YEp24, YCp50, etc.), a phage DNA (Charon4A, Charon21A, EMBL3, EMBL4, λgt10, λgt11, λZAP, etc.), an animal viral vector (retrovirus, adenovirus, vaccinia virus, etc.), or an insect viral vector (baculovirus, etc.). Since the expression vector has different levels of expression and modification of proteins depending on the host cell, it is desirable to select and use the host cell most suitable for the purpose.

The transformant provided in the present invention may be constructed by introducing the expression vector provided in the present invention into a host and transforming the host, and may be used to produce the peptide by expressing the polynucleotide included in the expression vector. The transformation may be performed by various methods, and is not particularly limited as long as it may produce the peptide. In this case, the transformation may be performed using a CaCl₂ precipitation method, a Hanahan method whose efficiency is enhanced by using a reducing substance called dimethyl sulfoxide (DMSO) in the CaCl₂ precipitation method, electroporation, a calcium phosphate precipitation method, a protoplast fusion method, a stirring method using silicon carbide fibers, an *Agrobacterium-mediated* transformation method, a transformation method using PEG, dextran sulfate, Lipofectamine, a drying/inhibition-mediated transformation method, and the like. In addition, the host used to construct the transformant is also not particularly limited as long as it may produce the peptide, and may be a bacterial cell such as *Escherichia coli, Streptomyces, Salmonella typhimurium,* or the like; a yeast cell such as *Saccharomyces cerevisiae, Schizosaccharomyces pombe,* or the like; a fungal cell such as *Pichia pastoris* or the like; an insect cell such as *Drosophila, Spodoptera* Sf9 cells, or the like; an animal cell such as CHO, COS, NSO, 293, Bow melanoma cells, or the like; or a plant cell.

The transformant may also be used in a method of producing a peptide including the amino acids set forth in SEQ ID NO: 1 of the present invention. Specifically, the method of producing a peptide including the amino acids set forth in SEQ ID NO: 1 of the present invention may include (a) culturing the transformant to obtain a culture; and (b) recovering the peptide of the present invention from the culture.

The term "culturing" of the present invention refers to a method of growing microorganisms under appropriately artificially controlled environmental conditions. In the present invention, the method of culturing the transformant may be performed using a method well-known in the art. Specifically, the culturing is not particularly limited as long as it may express and produce a peptide including the amino acids set forth in SEQ ID NO: 1 of the present invention, and may be continuously performed in a batch process or a fed batch or repeated fed batch process.

The medium used for culture should be a conventional medium containing a suitable carbon source, nitrogen source, amino acids, vitamins, and the like, and satisfy the requirements of a specific strain in an appropriate manner while controlling the temperature, pH, and the like under aerobic conditions. The carbon sources that may be used include a mixed sugar of glucose and xylose as the main carbon source, and also include sugars and carbohydrates such as sucrose, lactose, fructose, maltose, starch, and cellulose; oils and fats such as soybean oil, sunflower oil, castor oil, coconut oil, and the like; fatty acids such as palmitic acid, stearic acid, and linoleic acid; alcohols such as glycerol and ethanol; and organic acids such as acetic acid. These substances may be used alone or as in combination. Inorganic nitrogen sources such as ammonia, ammonium sulfate, ammonium chloride, ammonium acetate, ammonium phosphate, ammonium carbonate, and ammonium nitrate; organic nitrogen sources such as amino acids such as glutamic acid, methionine, and glutamine, and peptone, NZ-amine, a meat extract, a yeast extract, a malt extract, a corn steep liquor, a casein hydrolysate, fish or decomposition products thereof, defatted soybean cake or decomposition products thereof may be used as the nitrogen sources. These nitrogen sources may be used alone or in combination thereof. The medium may include monopotassium phosphate, dipotassium phosphate, and the corresponding sodium-containing salts as the phosphorus sources. The phosphorus sources that may be used include potassium dihydrogen phosphate or dipotassium hydrogen phosphate or the corresponding sodium-containing salts. In addition, sodium chloride, calcium chloride, iron chloride, magnesium sulfate, iron sulfate, manganese sulfate, calcium carbonate, and the like may be used as the inorganic compounds. Finally, in addition to the above substances, essential growth substances such as amino acids and vitamins may be used.

Also, suitable precursors may be used in the culture medium. The above-described raw materials may be added to the culture in a batch, fed-batch or continuous manner in an appropriate manner during a culture process, but the present invention is not particularly limited thereto. The pH of the culture may be adjusted using basic compounds such as sodium hydroxide, potassium hydroxide, and ammonia, or acid compounds such as phosphoric acid or sulfuric acid in an appropriate manner.

**In** addition, antifoaming agents such as fatty acid polyglycol esters may be used to suppress foaming. Oxygen or oxygen-containing gas (*e.g.,* air) is injected into the culture to maintain an aerobic state. The temperature of the culture is usually 27°C to 37°C, preferably 30°C to 35°C. The culturing is continued until the maximum production of the peptide is obtained. This is usually achieved in 10 to 100 hours.

Additionally, the recovering of the peptide from the culture may be performed by a method known in the art. Specifically, the recovery method is not particularly limited as long as it may be used to recover the produced peptide. Preferably, methods such as centrifugation, filtration, extraction, spraying, drying, evaporation, precipitation, crystallization, electrophoresis, differential dissolution (e.g., ammonium sulfate precipitation), chromatography (e.g., ion exchange, affinity, hydrophobicity, and size exclusion), and the like may be used.

The pharmaceutical composition of the present invention may be prepared in the form of a pharmaceutical composition for treating a heart disease caused by oxidative stress in cardiomyocytes or a skin disease caused by oxidative stress i dermal fibroblasts, which further includes an appropriate carrier (a natural or non-natural carrier), excipient or diluent commonly used in the preparation of pharmaceutical compositions as well as the peptide. Specifically, the pharmaceutical composition may be formulated and used in the form of a sterile injectable solution that may be administered to a site in which either heart disease or skin disease has been induced according to a conventional method. **In** the present invention, carriers, excipients and diluents that may be included in the pharmaceutical composition include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, mineral oil, collagen, and the like. When formulating, the composition may be prepared using commonly used diluents or excipients such as fillers, bulking agents, binders, wetting agents, disintegrating agents, surfactants, and the like. In particular, sterilized aqueous solutions, non-aqueous solvents, suspending agents, emulsifying agents, lyophilized preparations, suppositories, ointments (for example, dental pulp liners, etc.), and the like may be included. Propylene glycol, polyethylene glycol, vegetable oils such as olive oil, injectable esters such as ethyl oleate, and the like may be used as the non-aqueous solvents and suspending agents. Witepsol, Macrogol, Tween 61, cacao butter, laurin butter, glycerogelatin, and the like may be used as bases for suppositories.

The content of the peptide included in the pharmaceutical composition of the present invention is not particularly limited, but the peptide may be included in an amount of 0.0001 to 50% by weight, more preferably 0.01 to 20% by weight, based on the total weight of the final composition.

The pharmaceutical composition of the present invention may be administered in a pharmaceutically effective amount. Here, the term "pharmaceutically effective amount" used in the present invention refers to an amount sufficient to treat or prevent a disease at a reasonable benefit/risk ratio applicable for medical treatment or prevention, and the effective dosage level may be determined depending on the factors including the severity of a disease, the activity of a drug, the age, weight, health, and sex of a patient, the sensitivity of the patient to the drug, the administration time, administration route and excretion rate of the composition used in the present invention, the treatment period, drugs used in combination or simultaneously with the composition used in the present invention, and other factors well known in the medical field. The pharmaceutical composition of the present invention may be administered alone or in combination with a known pharmaceutical composition for treating a heart disease caused by oxidative stress in cardiomyocytes or a skin disease caused by oxidative stress in dermal fibroblasts. It is important to administer an amount that can achieve the maximum effect with the minimum amount without any side effects in consideration of all of the above factors.

The dose of the pharmaceutical composition of the present invention may be determined by those skilled in the art in consideration of the purpose of use, the severity of a disease, the age, weight, sex, and medical history of a patient, or the type of substance used as the active ingredient. For example, the pharmaceutical composition of the present invention may be administered to an adult at approximately 0.1 ng to approximately 100 mg/kg, preferably 1 ng to approximately 10 mg/kg, and the frequency of administration of the composition of the present invention is not particularly limited, but the composition may be administered once a day or administered several times in divided doses. The dose is not intended to limit the scope of the present invention in any way.

In another aspect, the present invention provides a method of treating a heart disease caused by oxidative stress in cardiomyocytes or a skin disease caused by oxidative stress in dermal fibroblasts, which includes administering a pharmaceutically effective amount of the pharmaceutical composition to a subject other than humans suffering from a heart disease caused by oxidative stress in cardiomyocytes or a skin disease caused by oxidative stress in dermal fibroblasts.

The term "subject" of the present invention may include, without limitation, mammals including mice, livestock, and the like, which require treatment for a heart disease caused by oxidative stress in cardiomyocytes or a skin disease caused by oxidative stress in dermal fibroblasts, but excludes humans from among subjects suffering from the above diseases.

The route of administration of the pharmaceutical composition of the present invention for treating a heart disease caused by oxidative stress in cardiomyocytes or a skin disease caused by oxidative stress in dermal fibroblasts may be administered via any general route as long as it may reach the target tissue. The pharmaceutical composition of the present invention may be administered via oral administration, transdermal administration, intramuscular injection, intravenous injection, subcutaneous injection, respiratory administration, rectal administration, or administration to an affected area, and the like depending on the purpose, but the present invention is not particularly limited thereto.

The "oral administration (intraoral administration)" is a process of taking a drug through the mouth. In this case, drugs in the form of pills, capsules, or liquids may be generally used as the drug. The "transdermal administration" is a process of applying or attaching a drug to the skin. In this case, the drug may be used in the form of creams, gels, patches, or the like. The "intramuscular injection" is a process of directly injecting a drug into muscle tissue, and may be mainly performed using a syringe and needle. The "intravenous injection" is a process of directly injecting a drug into blood vessels, and may be performed using a syringe and needle or an intravenous cannula. The "subcutaneous injection" is a process of injecting a drug into fatty tissue under the skin, and may be used to administer drugs such as insulin. The "respiratory administration" is a process of allowing a drug to reach the respiratory tract through the mouth or nose. In this case, the drug may be used in the form of an inhaler, gas, or vapor. The "rectal administration" is a process of injecting a drug into the rectum. In this case, the drug may be used in the form of rectal medications, rectal instillations, and the like. The "administration to an affected site" is a process of directly applying a drug to a site requiring treatment. In this case, the drug may be used in the form of creams, gels, or patches, depending on the characteristics of the disease.

In yet another aspect, the present invention provides a quasi-drug composition for preventing or ameliorating a heart disease caused by oxidative stress in cardiomyocytes or a skin disease caused by oxidative stress in dermal fibroblasts, which includes the peptide.

The term "amelioration" used in the present invention refers to any action that at least reduces parameters associated with the condition being treated, for example, the severity of a symptom.

In the present invention, the amelioration may be interpreted to refer to all actions that improve or beneficially change the symptoms of a heart disease caused by oxidative stress in cardiomyocytes or a skin disease caused by oxidative stress in dermal fibroblasts by administering the pharmaceutical composition including the peptide of the present invention as an active ingredient to a subject in need of treatment of a heart disease caused by oxidative stress in cardiomyocytes or a skin disease caused by oxidative stress in dermal fibroblasts in order to facilitate the scavenging of reactive oxygen species in the cardiomyocytes or dermal fibroblasts.

The term "quasi-drug" used in the present invention refers to products that have a milder effect than drugs among the products that are used for the purpose of diagnosing, treating, ameliorating, alleviating, managing or preventing diseases of humans or animals. For example, according to the Pharmaceutical Affairs Act, quasi-drugs are products other than those used for the purpose of drugs, and include fiber and rubber products used for the treatment or prevention of diseases of humans or animals, those that have a mild or no direct effect on the human body and are not instruments or machines and those similar thereto, and bactericides and insecticides for preventing infectious diseases.

In the present invention, the type or formulation of the quasi-drug composition including the peptide is not particularly limited, but may be, for example, an ointment, a patch, a powder, a gel, a spray, a tablet, or a spray.

In yet another aspect, the present invention provides a health functional food composition for preventing or ameliorating a heart disease caused by oxidative stress in cardiomyocytes or a skin disease caused by oxidative stress in dermal fibroblasts, which includes the peptide.

The term "food" used in the present invention includes meat, sausage, bread, chocolate, candies, snacks, confectioneries, pizza, ramen, other noodles, gum, dairy products including ice cream, various soups, beverages, tea, drinks, alcoholic beverages, vitamin complexes, health functional foods, health foods, and the like. In this case, the food includes all foods in the conventional sense.

The health functional food is the same term as food for special health use (FoSHU), and refers to food with high medical and healthcare effects that is processed to efficiently exhibit a bioregulatory function in addition to nutritional supply. Here, the term "functional" means regulating nutrients for the structure and function of the human body or obtaining a useful effect for health purposes such as physiological effects and the like. The food of the present invention may be manufactured by a method commonly used in the art, and may be manufactured by adding raw materials and ingredients commonly added in the art during the manufacturing process. Also, the formulation of the food may be manufactured without limitation as long as it is a formulation recognized as food. The food composition of the present invention may be manufactured in various forms of formulations. In this case, unlike general drugs, the food composition has the advantage of having no side effects that may occur when taking drugs for a long time because food is used as a raw material, and exhibits excellent portability. Thus, the food of the present invention may be consumed as a supplement to enhance the effect of preventing or ameliorating a heart disease caused by oxidative stress in cardiomyocytes or a skin disease caused by oxidative stress in dermal fibroblasts.

The health food refers to a food that has a more active health maintenance or promotion effect than general food, and the health supplement food refers to food for the purpose of health supplementation. In some cases, the terms "health functional food," "health food," and "health supplement food" may be used interchangeably.

Specifically, the health functional food refers to a food product manufactured by adding the peptide of the present invention to food materials such as beverages, teas, spices, gums, confectioneries, and the like or manufactured in the form of capsules, powder, suspensions, and the like. When consumed, the health functional food has a specific health effect; however, unlike general drugs, it has the advantage of having no side effects that may occur when taking drugs for a long time because food is used as a raw material.

Since the food composition of the present invention may be consumed on a daily basis, the food composition may be expected to be highly effective in preventing or ameliorating a heart disease caused by oxidative stress in cardiomyocytes or a skin disease caused by oxidative stress in dermal fibroblasts, and thus may be used very usefully.

The food composition may further include a physiologically acceptable carrier. In this case, the type of carrier is not particularly limited, and any carrier commonly used in the art may be used.

Also, the food composition may include additional ingredients commonly used in food compositions to improve odor, taste, appearance, and the like. For example, the food composition may include vitamins A, C, D, E, B1, B2, B6, B12, niacin, biotin, folate, pantothenic acid, and the like. Also, the food composition may include minerals such as zinc (Zn), iron (Fe), calcium (Ca), magnesium (Mg), manganese (Mn), copper (Cu), chromium (Cr), and the like. In addition, the food composition may include amino acids such as lysine, tryptophan, cysteine, valine, and the like.

In addition, the food composition may include food additives such as preservatives (potassium sorbate, sodium benzoate, salicylic acid, sodium dehydroacetate, etc.), bactericides (bleaching powder and highly bleaching powder, sodium hypochlorite, etc.), antioxidants (butylated hydroxyanisole (BHA), butylated hydroxytoluene (BHT), etc.), dyes (tar color, etc.), colorants (sodium nitrite, sodium nitrite, etc.), bleaching agents (sodium sulfite), seasonings (MSG, monosodium glutamate, etc.), sweeteners (dulcin, cyclamate, saccharin, sodium, etc.), flavorings (vanillin, lactones, etc.), leavening agents (alum, D-potassium hydrogen tartrate, etc.), reinforcing agents, emulsifiers, thickeners (gelling agents), film-forming agents, gum bases, foam suppressants, solvents, improving agents, and the like. The additives may be selected and used in an appropriate amount depending on the type of food.

The peptide of the present invention may be added as is or used together with other foods or food ingredients, and may be used appropriately according to a conventional method. The mixing amount of the active ingredient may be appropriately determined according to the purpose of use (prevention, health, or therapeutic treatment). In general, when manufacturing a food or beverage, the food composition of the present invention may be added to the food or beverage in an amount of 50 parts by weight or less, specifically 20 parts by weight or less. However, when consumed for a long time for health and hygiene purposes, the active ingredient may be included in an amount below the above range. Also, since there is no problem in terms of safety, the active ingredient may also be used in an amount above the above range.

As an example, the food composition of the present invention may be used as a health beverage composition. In this case, the health beverage composition may contain various flavoring agents, natural carbohydrates, or the like as additional ingredients like conventional beverages. The above-mentioned natural carbohydrates may include monosaccharides such as glucose and fructose; disaccharides such as maltose and sucrose; polysaccharides such as dextrin and cyclodextrin; sugar alcohols such as xylitol, sorbitol, erythritol, and the like. The sweetener may include a natural sweetener such as thaumatin and a stevia extract; a synthetic sweetener such as saccharin and aspartame; and the like. The proportion of the natural carbohydrate may be generally approximately 0.01 to 0.04 g, specifically approximately 0.02 to 0.03 g per 100 mL of the health beverage composition of the present invention.

In addition to the above ingredients, the health beverage composition may contain various nutrients, vitamins, electrolytes, flavoring agents, coloring agents, pectic acid, salts of pectic acid, alginic acid, salts of alginic acid, organic acids, protective colloid thickeners, pH regulators, stabilizers, preservatives, glycerin, alcohol, carbonating agents, or the like. In addition, the health beverage composition may contain fruit pulp for manufacturing natural fruit juice, fruit juice beverages, or vegetable beverages. These ingredients may be used alone or in combination. The proportion of these additives is not particularly important, but is generally selected in the range of 0.01 to 0.1 parts by weight based on 100 parts by weight of the health beverage composition of the present invention.

The food composition of the present invention may include the peptide of the present invention in various percentages by weight as long as it can exhibit the effect of preventing or improving a heart disease caused by oxidative stress in cardiomyocytes or a skin disease caused by oxidative stress in dermal fibroblasts. Specifically, the food composition may include the peptide of the present invention in an amount of 0.00001 to 100% by weight or 0.01 to 80% by weight based on the total weight of the food composition, but the present invention is not limited thereto.

### [Advantageous Effects]

The present invention can provide a composition for scavenging intracellular reactive oxygen species, which includes a CPNE7 protein or a peptide derived from the CPNE7 protein.

Another object of the present invention is to provide a polynucleotide encoding the peptide.

Still another object of the present invention is to provide an expression vector including the polynucleotide.

Yet another object of the present invention is to provide a pharmaceutical composition for preventing or treating a heart disease caused by oxidative stress in cardiomyocytes or a skin disease caused by oxidative stress in dermal fibroblasts, which includes the peptide.

Yet another object of the present invention is to provide a quasi-drug composition for preventing or ameliorating a heart disease caused by oxidative stress in cardiomyocytes or a skin disease caused by oxidative stress in dermal fibroblasts, which includes the peptide.

Yet another object of the present invention is to provide a health functional food composition for preventing or ameliorating a heart disease caused by oxidative stress in cardiomyocytes or a skin disease caused by oxidative stress in dermal fibroblasts, which includes the peptide.

The effects of the present invention are not limited to those mentioned above, and other effects not mentioned herein will be clearly understood by those skilled in the art to which the present invention pertains from the description below.

### [Description of Drawings]

FIG. 1 is a graph comparing the level of reactive oxygen species using the cell permeant reagent 2',7'-dichlorofluorescin diacetate (DCFDA, also known as H2DCFDA, DCFH-DA, and DCFH), showing that when Cpne7 is reduced through ShCpne7 transformation, the level of reactive oxygen species increases approximately 2 times or higher compared to the control.
FIG. 2 shows the results of comparing the level of reactive oxygen species using the cell permeant reagent 2',7'-dichlorofluorescin diacetate (DCFDA, also known as H2DCFDA, DCFH-DA, and DCFH).
FIG. 3 shows the results of measuring the amount of living cells using highly sensitive water-soluble tetrazolium salt (WST-8).
FIG. 4 shows the results of evaluating the effects of increased reactive oxygen species on odontoblasts in Cpne7 knockout mice (DNA damage) and comparatively confirming the degree of DNA damage through immunofluorescence staining with γ-H2A.X which is a DNA damage marker protein.
FIG. 5 shows the results of evaluating the effect of increased reactive oxygen species on odontoblasts in Cpne7 knockout mice (cell aging) and comparing the degree of cell aging through staining with beta-galactosidase (B-gal) which is a cell aging marker enzyme.
FIG. 6 shows the results of evaluating the effects (histomorphological) of increased reactive oxygen species on odontoblasts in Cpne7 knockout mice.
FIG. 7 shows the results of confirming the reactive oxygen species scavenging ability of a CPNE7 recombinant protein (rCPNE7).
FIG. 8 shows the results of confirming the reactive oxygen species scavenging ability of the CPNE7 recombinant protein (rCPNE7) by comparing the level of reactive oxygen species using the cell permeant reagent 2',7'-dichlorofluorescin diacetate (DCFDA, also known as H2DCFDA, DCFH-DA, and DCFH).
FIG. 9 shows the results of confirming the reactive oxygen species scavenging ability of the CPNE7 recombinant protein (rCPNE7) by comparatively confirming the degree of DNA damage using immunofluorescence staining with γ-H2A.X which is a DNA damage marker protein.
FIG. 10 shows the results of confirming the reactive oxygen species scavenging ability of the CPNE7 recombinant protein (rCPNE7) by comparing the degree of cell aging through staining with beta-galactosidase (B-gal) which is a cell aging marker enzyme.
FIG. 11 shows the results of measuring the amount of living cells using highly sensitive water-soluble tetrazolium salt (WST-8) and confirming the cell viability of hDPCs according to the concentration of reactive oxygen species.
FIG. 12 shows the results of confirming the reactive oxygen species scavenging ability of a CPNE7 protein-derived functional peptide (Selcopintide) according to its concentration.
FIG. 13 shows the results of confirming the reactive oxygen species scavenging ability of the CPNE7 protein-derived functional peptide (Selcopintide) according to its concentration by comparing the level of reactive oxygen species using the cell permeant reagent 2',7'-dichlorofluorescin diacetate (DCFDA, also known as H2DCFDA, DCFH-DA, and DCFH).
FIG. 14 shows the results of confirming the reactive oxygen species scavenging ability of the CPNE7 protein-derived functional peptide (Selcopintide) according to its concentration based on the comparison of the level of reactive oxygen species through the mRNA levels of endogenous antioxidant enzymes in hDPC cells by Selcopintide using RT-PCR.
FIG. 15 shows the results of confirming the reactive oxygen species scavenging ability of Selcopintide by comparatively confirming the degree of DNA damage through immunofluorescence staining with γ-H2A.X which is a DNA damage marker protein.
FIG. 16 shows the results of measuring the amount of living cells using highly sensitive water-soluble tetrazolium salt (WST-8).
FIG. 17 shows the results of confirming the reactive oxygen species scavenging ability of Selcopintide in cardiomyocytes by treating cells with H₂O₂, which induces intracellular reactive oxygen species, and treating Selcopintide, and then confirming the cells under an optical microscope and measuring the amount of living cells using highly sensitive water-soluble tetrazolium salt (WST-8).
FIG. 18 shows the results of confirming the reactive oxygen species scavenging ability of Selcopintide in cardiomyocytes by comparing the degree of cell aging through staining with beta-galactosidase (B-gal) which is a cell aging marker enzyme.
FIG. 19 shows the results of confirming the reactive oxygen species scavenging ability of Selcopintide in dermal fibroblasts by measuring the amount of living cells using highly sensitive water-soluble tetrazolium salt (WST-8).
FIG. 20 shows the results confirming the reactive oxygen species scavenging ability of Selcopintide in dermal fibroblasts.

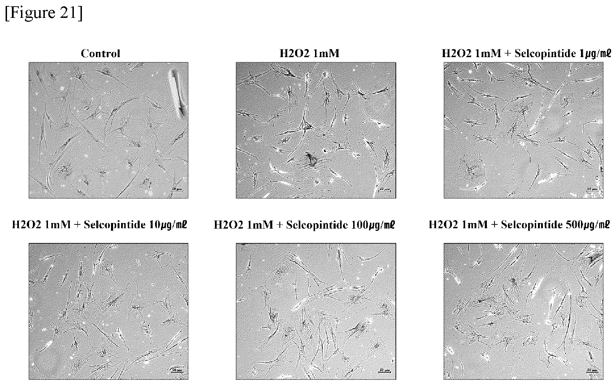

FIG. 21 shows the results of confirming the reactive oxygen species scavenging ability of Selcopintide in dermal fibroblasts by comparing the degree of cell aging through staining with beta-galactosidase (B-gal) which is a cell aging marker enzyme.

### [Mode for Invention]

The objects and effects of the present invention and the technical configurations for achieving them will become clearer with reference to the embodiments described in detail below with the accompanying drawings. In describing the present invention, when it is judged that a specific description of a known function or configuration may unnecessarily obscure the gist of the present invention, the detailed description thereof will be omitted. In addition, the terms described below are terms defined in consideration of the functions in the present invention, and these may vary depending on the intention or custom of the user or operator.

However, the present invention is not limited to the embodiments disclosed below, but can be implemented in various different forms. It should be understood that these embodiments are presented only to ensure that the disclosure of the present invention is complete and to fully inform those skilled in the art to which the present invention pertains of the scope of the invention, and the present invention is defined only by the scope of the claims. Therefore, the definition should be made based on the contents throughout the present specification.

Hereinafter, embodiments of the present invention will be described in more detail.

### Example 1: Comparison of reactive oxygen species levels using cell permeant reagent 2',7'-dichlorofluorescin diacetate (DCFDA, also known as H2DCFDA, DCFH-DA, and DCFH)

Human dental pulp cells (hDPCs) were cultured in an α-MEM medium containing 10% FBS under 37°C and 5% CO₂ conditions.

Next, the cultured hDPC cells were seeded into 60 mm culture dishes at a density of 4 x 10⁵ cells per dish, cultured for 24 hours, and then the cultured cells were transformed with the Shcon vector and ShCpne7 vector using a Lipofectamine Plus^{™} reagent.

The transformed hDPC cells were seeded again in a 96-well plate at a density of 3 x 10³ cells per well, cultured for 24 hours, and then treated with the DCFDA substance. 40 minutes later, the level of reactive oxygen species was measured at Ex/Em = 485/535 nm using a microplate reader. At this time, the untreated control and the Shcon vector were used as controls.

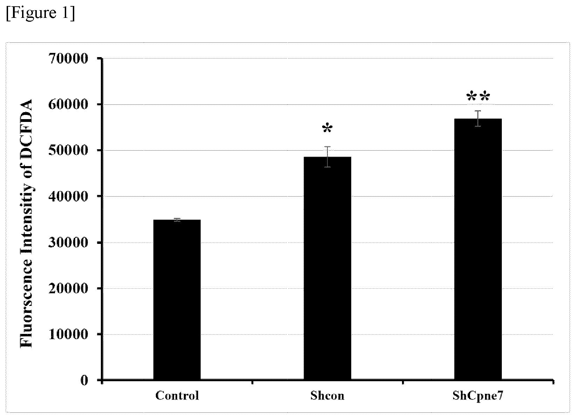

Referring to FIG. 1, it can be seen that when Cpne7 was reduced through ShCpne7 transformation, the level of reactive oxygen species increased approximately 2 times or higher compared to the control. Based on these results, it can be seen that Cpne7 is effective in maintaining the level of reactive oxygen species at a normal level in hDPC cells.

### Example 2: Comparison of reactive oxygen species levels using cell permeant reagent 2',7'-dichlorofluorescin diacetate (DCFDA, also known as H2DCFDA, DCFH-DA, and DCFH)

Each mandibular molar tissue section of 6-month-old mice in which the Cpne7 gene was knocked out was treated with the DCFDA substance, and the levels of reactive oxygen species were measured using a confocal microscope 40 minutes later. At this time, mandibular molars of 6-month-old BL6 mice were used as the control.

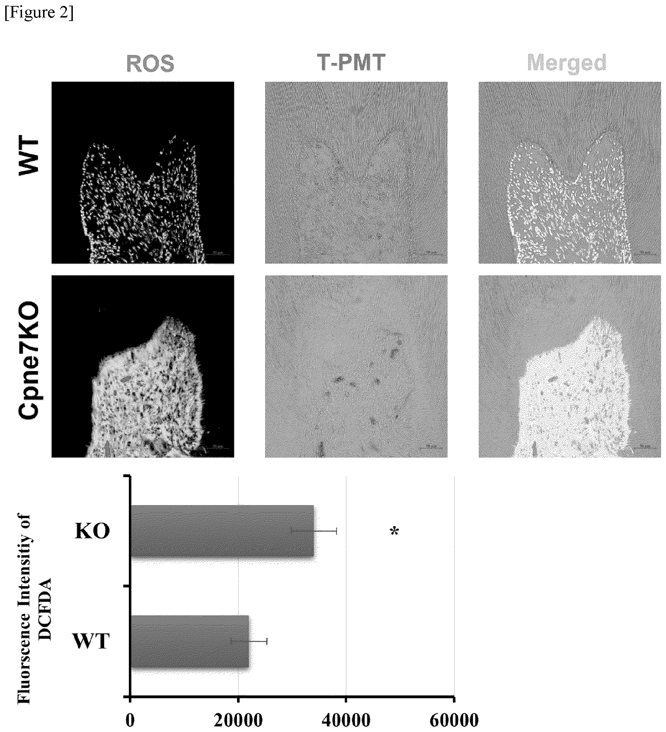

When the Cpne7 gene was knocked out, it was confirmed that the *in vivo* level of reactive oxygen species in odontoblasts increased compared to the control, similar to the *in vitro* results (FIG. 2).

From the above results, it was confirmed that Cpne7 is a factor that maintains the level of reactive oxygen species in the living body including odontoblasts at a normal level.

### Example 3: Verification of oxidative stress vulnerability upon Cpne7 knockdown using odontoblasts

Cultured hDPC cells were seeded into 96-well plates at a density of 3 x 10³ cells per well, cultured for 24 hours, and then treated with 50 uM and 100 uM hydrogen peroxide (H₂O₂) for 24 hours. The WST-8 solution was added to each well, reacted for one hour, and then absorbance was measured at 450 nm using a microplate reader and compared.

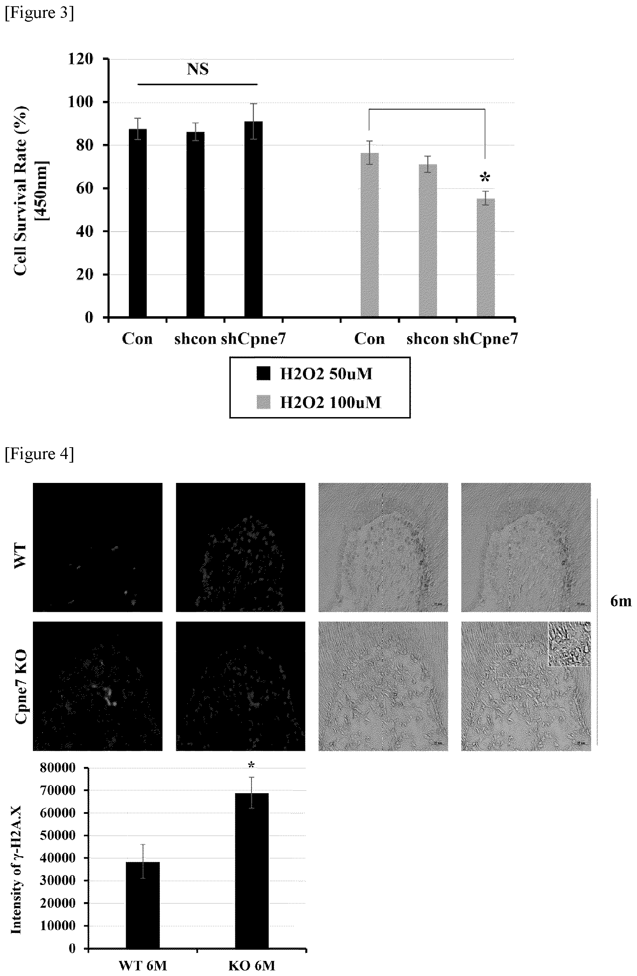

The measurement results showed that cell viability significantly decreased at a concentration of 100 uM H₂O₂ in the group in which the Cpne7 gene was knocked down (ShCpne7), compared to the control (FIG. 3). As a result, it can be seen that Cpne7 is involved in maintaining the level of reactive oxygen species at a normal level in hDPC cells.

### Example 4: Comparative confirmation of degree of DNA damage through immunofluorescence staining with γ-H2A.X which is a DNA damage marker protein

From the experimental results of FIGS. 1 and 2, it was confirmed that Cpne7 is involved in maintaining the normal level of reactive oxygen species, and in particular, reactive oxygen species increase when Cpne7 is knocked out. As a result, it was confirmed whether an increase in reactive oxygen species can increase DNA damage in odontoblasts.

Immunofluorescence staining using a γ-H2A.X antibody was performed on mandibular molar tissue sections from 6-month-old Cpne7 knockout mice to compare intensity. At this time, the mandibular molar tissue sections from 6-month-old BL6 mice were used as the control.

As can be seen in FIG. 4, it was confirmed that DNA damage accumulated in odontoblasts of the Cpne7 knockout mice with increased reactive oxygen species.

Based on the above results, it was analyzed that the increase in reactive oxygen species due to Cpne7 knockout induced DNA damage.

### Example 5: Comparison of the degree of cell aging through staining with beta-galactosidase (B-gal) which is a cell aging marker enzyme

From the experimental results of FIGS. 1, 2, and 4, it was confirmed that the increased reactive oxygen species induce DNA damage when Cpne7 is knocked out. As a result, it was confirmed whether induced DNA damage can promote cell aging.

Transformed hDPCs were seeded into 35 mm culture dishes at a density of 1 x 10⁵ cells and cultured for 24 hours. Then, cell differentiation was performed for up to 21 days while replacing the α-MEM medium containing 5% FBS, 50 ug/mL ascorbic acid, and 10 mM β-glycerophosphate every other day. Differentiated odontoblasts on days 0, 4, 7, 14, and 21 were fixed in 4% paraformaldehyde, and stained with B-gal for 24 hours in a 37°C incubator without CO₂.

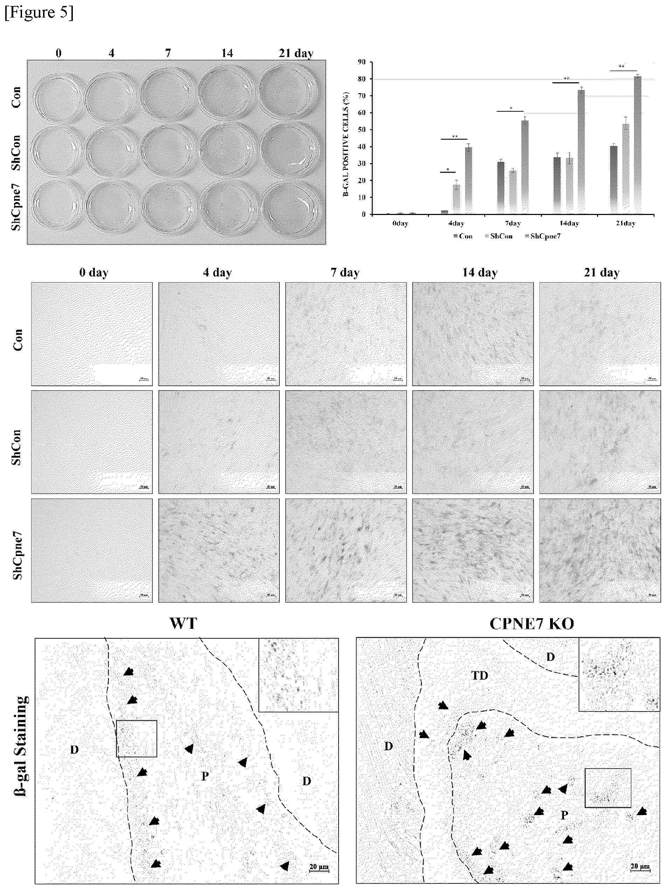

Also, B-gal staining was performed for 24 hours on mandibular molar tissue sections from 6-month-old Cpne7 knockout mice in which reactive oxygen species and DNA damage were confirmed. As shown in FIG. 5, it was confirmed that there were more B-gal positive cells in odontoblasts in which the Cpne7 gene was knocked down or knocked out compared to the control.

As a result, it was confirmed that the increase in reactive oxygen species due to the Cpne7 knockout induces DNA damage and promotes cell aging.

### Example 6: Histomorphological comparison of mandibular molars from 6- and 12-month-old Cpne7 knockout mice

Hematoxylin and Eosin (H&E) staining was performed on tissue sections of mandibular molars from 6- and 12-month-old Cpne7 knockout mice.

From the results of FIGS. 1, 2, 3, and 4, it was confirmed that the Cpne7 knockout odontoblasts induce DNA damage and promote cell aging through an increase in reactive oxygen species.

Histomorphological comparison of mandibular molars from the 6- and 12-month-old Cpne7 knockout mice revealed that the odontoblasts in 6-month-old Cpne7 knockout mandibular molars were irregularly arranged and also had abnormally shaped cell nuclei. Also, it was confirmed that pathological bone-like hard tissue was formed within the dental pulp tissue.

It was confirmed that more pathological bone-like hard tissue was formed within the dental pulp tissue in the 12-month-old Cpne7 knockout tooth tissue, and most odontoblasts died, and the condition of the remaining odontoblasts was unhealthy compared to the control.

As a result, it was confirmed that the increase in reactive oxygen species due to the Cpne7 knockout induces DNA damage and cell aging in odontoblasts, thereby causing a pathological condition.

### Example 7: Confirmation of cells under optical microscope after treatment with H₂O₂, which induces intracellular reactive oxygen species, and treatment with CPNE7 recombinant protein

hDPCs were seeded into 35 mm culture dishes at a density of 1 x 10⁵ cells, cultured for 24 hours, treated with H₂O₂ at a concentration of 5 uM, 100 uM, 1 mM, or 2 mM, respectively, and then treated with 100 ng/ml of a recombinant CPNE7 protein and cultured for 24 hours. After the culture was completed, the cultured cells were washed with PBS, and observed using an optical microscope. At this time, the hDPCs cultured without treating the recombinant CPNE7 protein (rCPNE7) were used as the control.

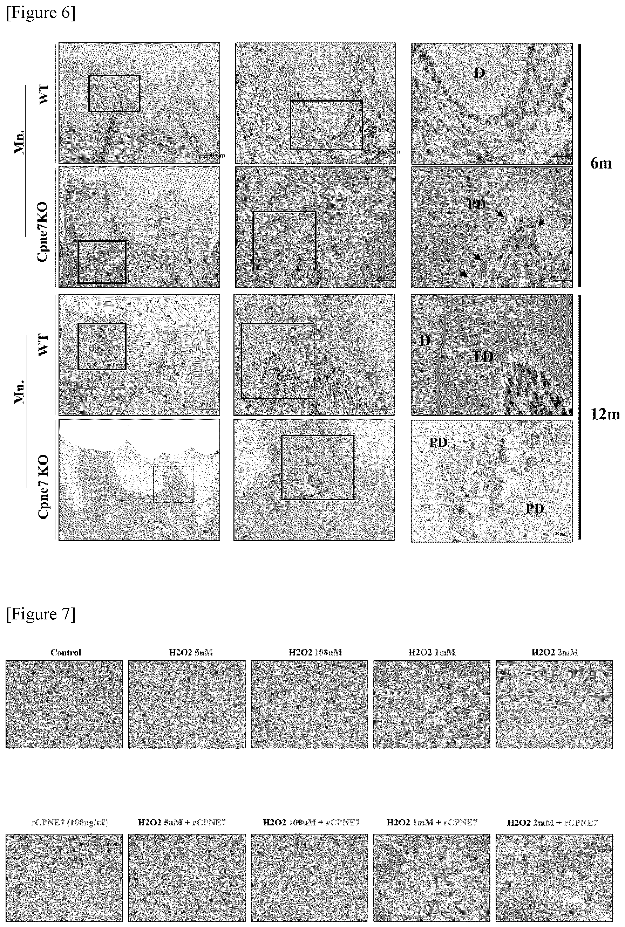

As can be seen in FIG. 7, no changes were observed at H₂O₂ concentrations below 100 uM, and cell death due to oxidative stress was observed at concentrations above 1 mM. On the other hand, cell viability was observed to be higher in the recombinant CPNE7 protein-treated group compared to the control. Based on these results, it was analyzed that the CPNE7 protein has a reactive oxygen species scavenging effect.

### Example 8: Comparison of reactive oxygen species levels using cell permeant reagent 2',7'-dichlorofluorescin diacetate (DCFDA, also known as H2DCFDA, DCFH-DA, and DCFH)

Cultured hDPCs were seeded into 35 mm culture dishes for a confocal microscope at a density of 1 x 10⁵ cells, cultured for 24 hours, and then treated with 1 mM H₂O₂ and treated with 100 ng/mL of rCPNE7. Then, images were taken for approximately one hour using a confocal microscope.

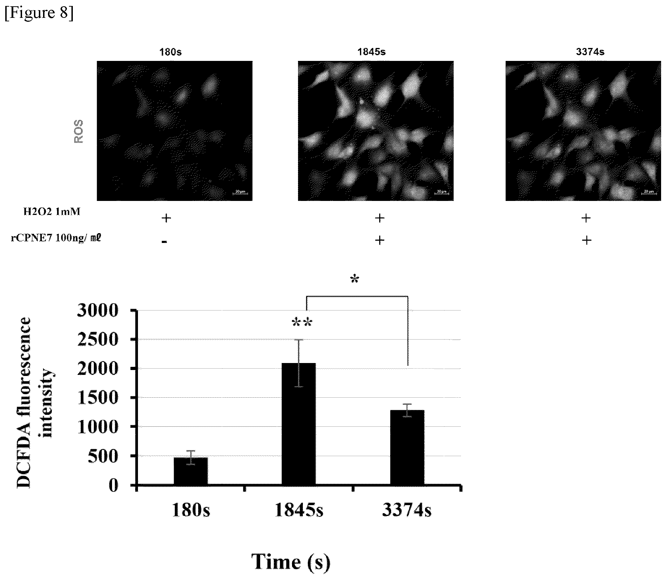

Referring to FIG. 8, after treatment with 1 mM H₂O₂, the amount of reactive oxygen species reached its maximum at approximately 30 minutes, then decreased and became significant at approximately one hour. Based on these results, it was confirmed that the CPNE7 protein has the potential to scavenge reactive oxygen species.

### Example 9: Comparison of DNA damage levels through immunofluorescence staining with γ-H2A.X which is a DNA damage marker protein

In FIG. 8, it was confirmed that the CPNE7 recombinant protein scavenges excessive reactive oxygen species in hDPCs, and thus the degree of DNA damage was confirmed.

Cultured hDPCs were seeded into 35 mm culture dishes for a confocal microscope at a density of 1 x 10⁵ cells, cultured for 24 hours, and then treated with 1 mM H₂O₂ and treated with 100 ng/mL of rCPNE7. After 24 hours, the cells were fixed with 4% paraformaldehyde.

Immunofluorescence staining using a γ-H2A.X antibody was performed on the fixed cells to compare intensity.

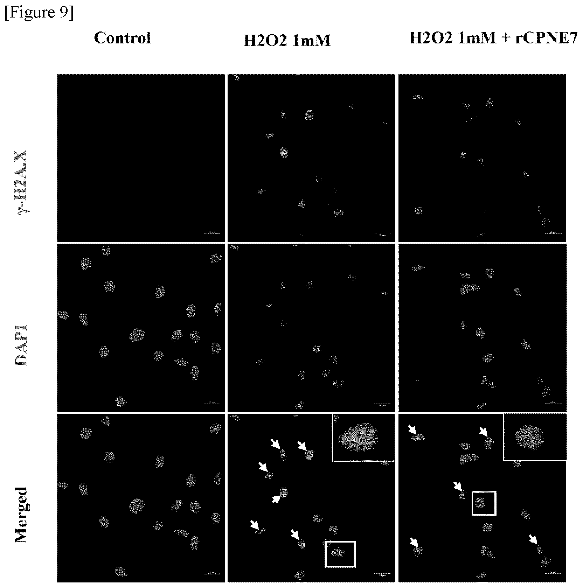

Referring to FIG. 9, the comparison results showed that a significant amount of DNA damage was induced in the group treated with 1 mM H₂O₂ to induce oxidative stress, whereas the accumulation of DNA damage was significantly reduced in the group treated with 100 ng/mL of the CPNE7 recombinant protein. Based on these results, it can be seen that the CPNE7 protein prevents or repairs DNA damage by scavenging excessive reactive oxygen species.

### Example 10: Comparison of degree of cell aging through staining with beta-galactosidase (B-gal) which is a cell aging marker enzyme

In FIGS. 8 and 9, it was confirmed that the CPNE7 recombinant protein prevents or repairs DNA damage by scavenging excessive reactive oxygen species in hDPCs, and the effect of the CPNE7 recombinant protein on cell aging was investigated.

For this purpose, cultured hDPCs were seeded into 6-well plates at a density of 1×10⁵ cells, cultured for 24 hours, and then treated with 1 mM H₂O₂ and treated with 100 ng/mL of rCPNE7. After 12 or 24 hours, the cells were fixed with 4% paraformaldehyde.

Next, B-gal staining was performed on the fixed cells for 24 hours to compare the degree of cell aging.

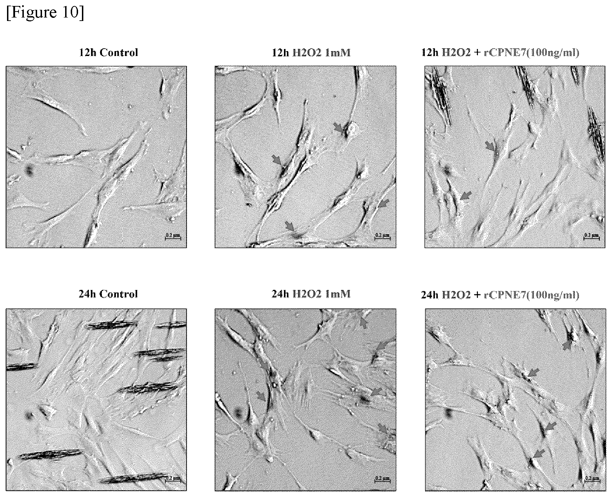

Referring to FIG. 10, it was confirmed that B-gal positive hDPC cells were identified 12 hours after treatment with 1 mM H₂O₂, and more B-gal positive hDPC cells were identified after 24 hours. On the other hand, it was confirmed that the B-gal positive hDPC cells were significantly reduced in the CPNE7 recombinant protein-treated group, compared to the H₂O₂-only-treated group. Based on these results, it was confirmed that the reactive oxygen species scavenging ability of the CPNE7 protein can inhibit cell aging by preventing or repairing DNA damage.

### Example 11: Measurement of amount of living cells using highly sensitive water-soluble tetrazolium salt (WST-8)

Cultured hDPC cells were seeded into 96-well plates at a density of 3 x 10³ cells per well, cultured for 24 hours, and then treated with hydrogen peroxide (H₂O₂) at concentrations of 0 uM (Control), 50 uM, 100 uM, 200 uM, 500 uM, 1 mM, and 2 mM for 24 hours. The WST-8 solution was added to each well and allowed to react for one hour. Then, the absorbance was measured at 450 nm using a microplate reader and compared.

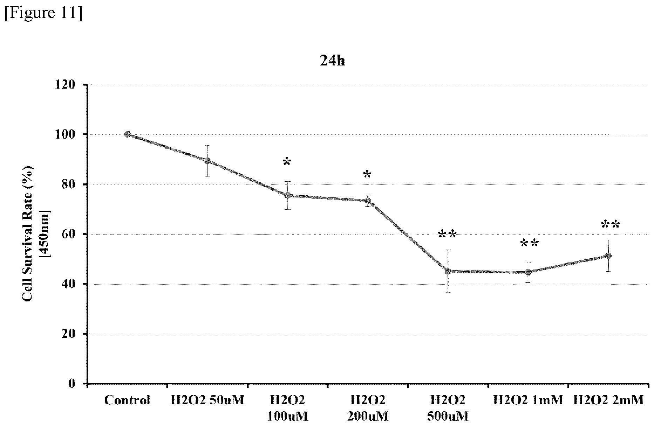

As shown in FIG. 11, it was confirmed that cell viability decreased as the concentration of H₂O₂ increased, and similar cell viability was observed at a H₂O₂ concentration of 500 uM or higher. Thereafter, a concentration of 1 mM H₂O₂ was selected as the concentration that induces oxidative stress.

### Example 12: Synthesis of peptides for scavenging intracellular reactive oxygen species

The present inventors synthesized a peptide (SEQ ID NO: 1) exhibiting an intracellular reactive oxygen species scavenging effect using a 9-fluorenylmethyloxycarbonyl (Fmoc) method, and synthesized peptides of each group by substituting amino acids of the synthesized peptide (Tables 1 to 12).
N-KYQRRKKNKY-C (SEQ ID NO: 1)

First, the peptides of Group 1 were synthesized by substituting the peptide of SEQ ID NO: 1 or amino acids 5 to 7 of the peptide of SEQ ID NO: 1 with lysine or arginine (Table 1).

**[Table 1]**

| Peptides of Group 1 | |
|---|---|
| SEQ ID NO: | Amino acid sequence (N-C) |
| 1 | KYQRRKKNKY |
| 2 | KYQRRKRNKY |
| 3 | KYQRRRKNKY |
| 4 | KYQRRRRNKY |
| 5 | KYQRKKKNKY |
| 6 | KYQRKRKNKY |
| 7 | KYQRKKRNKY |
| 8 | KYQRKRRNKY |

Next, the peptides of Group 2 were synthesized by substituting amino acids 5 to 7 of the peptide of SEQ ID NO: 1 with lysine or arginine and substituting amino acid 8 with serine (Table 2).

**[Table 2]**

| Peptides of Group 2 | |
|---|---|
| SEQ ID NO: | Amino acid sequence (N-C) |
| 9 | KYQRRKKSKY |
| 10 | KYQRRKRSKY |
| 11 | KYQRRRKSKY |
| 12 | KYQRRRRSKY |
| 13 | KYQRKKKSKY |
| 14 | KYQRKRKSKY |
| 15 | KYQRKKRSKY |
| 16 | KYQRKRRSKY |

Next, the peptides of Group 3 were synthesized by substituting amino acids 5 to 7 of the peptide of SEQ ID NO: 1 with lysine or arginine and substituting amino acid 9 with tyrosine (Table 3).

**[Table 3]**

| Peptides of Group 3 | |
|---|---|
| SEQ ID NO: | Amino acid sequence (N-C) |
| 17 | KYQRRKKNYK |
| 18 | KYQRRKRNYK |
| 19 | KYQRRRKNYK |
| 20 | KYQRRRRNYK |
| 21 | KYQRKKKNYK |
| 22 | KYQRKRKNYK |
| 23 | KYQRKKRNYK |
| 24 | KYQRKRRNYK |

Next, the peptides of Group 4 were synthesized by substituting amino acids 5 to 7 of the peptide of SEQ ID NO: 1 with lysine or arginine, substituting amino acid 8 with serine, substituting amino acid 9 with tyrosine, and substituting amino acid 10 with lysine (Table 4).

**[Table 4]**

| Peptides of Group 4 | |
|---|---|
| SEQ ID NO: | Amino acid sequence (N-C) |
| 25 | KYQRRKKSYK |
| 26 | KYQRRKRSYK |
| 27 | KYQRRRKSYK |
| 28 | KYQRRRRSYK |
| 29 | KYQRKKKSYK |
| 30 | KYQRKRKSYK |
| 31 | KYQRKKRSYK |
| 32 | KYQRKRRSYK |

Next, the peptides of Group 5 were synthesized by substituting amino acid 3 of the peptide of SEQ ID NO: 1 with arginine, substituting amino acid 4 with glutamine, and substituting amino acids 5 to 7 with lysine or arginine (Table 5).

**[Table 5]**

| Peptides of Group 5 | |
|---|---|
| SEQ ID NO: | Amino acid sequence (N-C) |
| 33 | KYRQRKKNKY |
| 34 | KYRQRKRNKY |
| 35 | KYRQRRKNKY |
| 36 | KYRQRRRNKY |
| 37 | KYRQKKKNKY |
| 38 | KYRQKRKNKY |
| 39 | KYRQKKRNKY |
| 40 | KYRQKRRNKY |

Next, the peptides of Group 6 were synthesized by substituting amino acid 3 of the peptide of SEQ ID NO: 1 with arginine, substituting amino acid 4with glutamine, substituting amino acids 5 to 7 with lysine or arginine, and substituting amino acid 8 with serine (Table 6).

**[Table 6]**

| Peptides of Group 6 | |
|---|---|
| SEQ ID NO: | Amino acid sequence (N-C) |
| 41 | KYRQRKKSKY |
| 42 | KYRQRKRSKY |
| 43 | KYRQRRKSKY |
| 44 | KYRQRRRSKY |
| 45 | KYRQKKKSKY |
| 46 | KYRQKRKSKY |
| 47 | KYRQKKRSKY |
| 48 | KYRQKRRSKY |

Next, the peptides of Group 7 were synthesized by substituting amino acid 3 of the peptide of SEQ ID NO: 1 with arginine, substituting amino acid 4 with glutamine, substituting amino acids 5 to 7 with lysine or arginine, substituting amino acid 9 with tyrosine, and substituting amino acid 10 with lysine (Table 7).

**[Table 7]**

| Peptides of Group 7 | |
|---|---|
| SEQ ID NO: | Amino acid sequence (N-C) |
| 49 | KYRQRKKNYK |
| 50 | KYRQRKRNYK |
| 51 | KYRQRRKNYK |
| 52 | KYRQRRRNYK |
| 53 | KYRQKKKNYK |
| 54 | KYRQKRKNYK |
| 55 | KYRQKKRNYK |
| 56 | KYRQKRRNYK |

Next, the peptides of Group 8 were synthesized by substituting amino acid 3 of the peptide of SEQ ID NO: 1 with arginine, substituting amino acid 4 with glutamine, substituting amino acids 5 to 7 with lysine or arginine, substituting amino acid 8 with serine, substituting amino acid 9 with tyrosine, and substituting amino acid 10 with lysine (Table 8).

**[Table 8]**

| Peptides of Group 8 | |
|---|---|
| SEQ ID NO: | Amino acid sequence (N-C) |
| 57 | KYRQRKKSYK |
| 58 | KYRQRKRSYK |
| 59 | KYRQRRKSYK |
| 60 | KYRQRRRSYK |
| 61 | KYRQKKKSYK |
| 62 | KYRQKRKSYK |
| 63 | KYRQKKRSYK |
| 64 | KYRQKRRSYK |

Next, the peptides of Group 9 were synthesized by substituting amino acid 3 of the peptide of SEQ ID NO: 1 with lysine, substituting amino acid 4 with glutamine, and substituting amino acids 5 to 7 with lysine or arginine (Table 9).

**[Table 9]**

| Peptides of Group 9 | |
|---|---|
| SEQ ID NO: | Amino acid sequence (N-C) |
| 65 | KYKQRKKNKY |
| 66 | KYKQRKRNKY |
| 67 | KYKQRRKNKY |
| 68 | KYKQRRRNKY |
| 69 | KYKQKKKNKY |
| 70 | KYKQKRKNKY |
| 71 | KYKQKKRNKY |
| 72 | KYKQKRRNKY |

Next, the peptides of Group 10 were synthesized by substituting amino acid 3 of the peptide of SEQ ID NO: 1 with lysine, substituting amino acid 4 with glutamine, substituting amino acids 5 to 7 with lysine or arginine, and substituting amino acid 8 with serine (Table 10).

**[Table 10]**

| Peptides of Group 10 | |
|---|---|
| SEQ ID NO: | Amino acid sequence (N-C) |
| 73 | KYKQRKKSKY |
| 74 | KYKQRKRSKY |
| 75 | KYKQRRKSKY |
| 76 | KYKQRRRSKY |
| 77 | KYKQKKKSKY |
| 78 | KYKQKRKSKY |
| 79 | KYKQKKRSKY |
| 80 | KYKQKRRSKY |

Next, the peptides of Group 11 were synthesized by substituting amino acid 3 of the peptide of SEQ ID NO: 1 with lysine, substituting amino acid 4 with glutamine, substituting amino acids 5 to 7 with lysine or arginine, substituting amino acid 9 with tyrosine, and substituting amino acid 10 with lysine (Table 11).

**[Table 11]**

| Peptides of Group 11 | |
|---|---|
| SEQ ID NO: | Amino acid sequence (N-C) |
| 81 | KYKQRKKNYK |
| 82 | KYKQRKRNYK |
| 83 | KYKQRRKNYK |
| 84 | KYKQRRRNYK |
| 85 | KYKQKKKNYK |
| 86 | KYKQKRKNYK |
| 87 | KYKQKKRNYK |
| 88 | KYKQKRRNYK |

Finally, the peptides of Group 12 were synthesized by substituting amino acid 3 of the peptide of SEQ ID NO: 1 with lysine, substituting amino acid 4 with glutamine, substituting amino acids 5 to 7 with lysine or arginine, substituting amino acid 8 with serine, substituting amino acid 9 with tyrosine, and substituting amino acid 10 with lysine (Table 12).

**[Table 12]**

| Peptides of Group 12 | |
|---|---|
| SEQ ID NO: | Amino acid sequence (N-C) |
| 89 | KYKQRKKSYK |
| 90 | KYKQRKRSYK |
| 91 | KYKQRRKSYK |
| 92 | KYKQRRRSYK |
| 93 | KYKQKKKSYK |
| 94 | KYKQKRKSYK |
| 95 | KYKQKKRSYK |
| 96 | KYKQKRRSYK |

### Example 13: Confirmation of concentration-dependent reactive oxygen species scavenging ability of CPNE7 protein-derived functional peptide (Selcopintide)

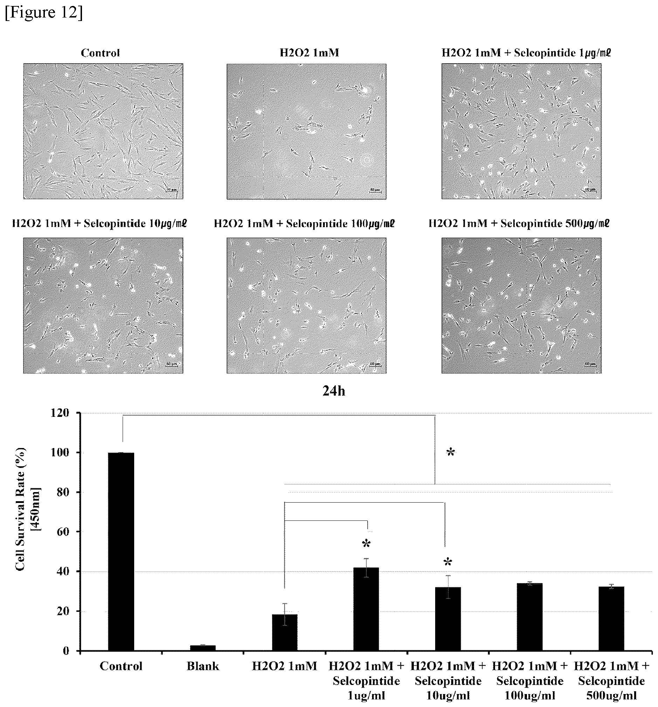

FIG. 12 shows the results of confirming cells under an optical microscope and measuring the amount of living cells using highly sensitive water-soluble tetrazolium salt (WST-8) after the cells are treated with H₂O₂, which induces intracellular reactive oxygen species, and treated with with Selcopintide (SEQ ID NO: 96).

Cultured hDPC cells were seeded into 6-well plates at a density of 1 x 10⁵ cells per well, cultured for 24 hours, and then treated with 1 mM hydrogen peroxide (H₂O₂) and treated with Selcopintide at concentrations of 1 ug/mL, 10 ug/mL, 100 ug/mL, and 500 ug/mL for 24 hours. Untreated hDPCs were used as the control.

In the 1 mM H₂O₂-treated group, significant apoptosis of odontoblasts was observed compared to the control. On the other hand, it was confirmed that cell viability was higher in the Selcopintide-treated groups compared to the H₂O₂-only-treated group. In particular, it was confirmed that cell viability was high in the 1 ug/mL and 10 ug/mL treatment groups (FIG. 12).

Cultured hDPC cells were seeded into 96-well plates at a density of 3 x 10³ cells per well, cultured for 24 hours, and then treated with 1 mM hydrogen peroxide (H₂O₂) and treated with Selcopintide at concentrations of 1 ug/mL, 10 ug/mL, 100 ug/mL, and 500 ug/mL for 24 hours. Untreated hDPCs were used as the control.

The WST-8 solution was added to each well and allowed to react for 1 hour, and then absorbance was measured at 450 nm using a microplate reader and compared. It was confirmed that cell viability was significantly reduced in the H₂O₂-only-treated group compared to the control, but cell viability was significantly higher in the 1 ug/mL and 10 ug/mL Selcopintide-treated groups compared to the H₂O₂-only-treated group (FIG. 12).

Based on the above results, it was confirmed that the CPNE7 protein-derived functional peptide (Selcopintide) has reactive oxygen species scavenging ability.

### Example 14: Confirmation of concentration-dependent reactive oxygen species scavenging ability of CPNE7 protein-derived functional peptide (Selcopintide) through comparison of reactive oxygen species levels using cell permeant reagent 2',7'-dichlorofluorescin diacetate (DCFDA, also known as H2DCFDA, DCFH-DA, and DCFH)

Cultured hDPCs were seeded into 35 mm culture dishes for a confocal microscope at a density of 1 x 10⁵ cells, cultured for 24 hours, and then treated with 1 mM H₂O₂ and treated with 10 ug/mL of Selcopintide. Thereafter, images were taken for approximately one hour using a confocal microscope.

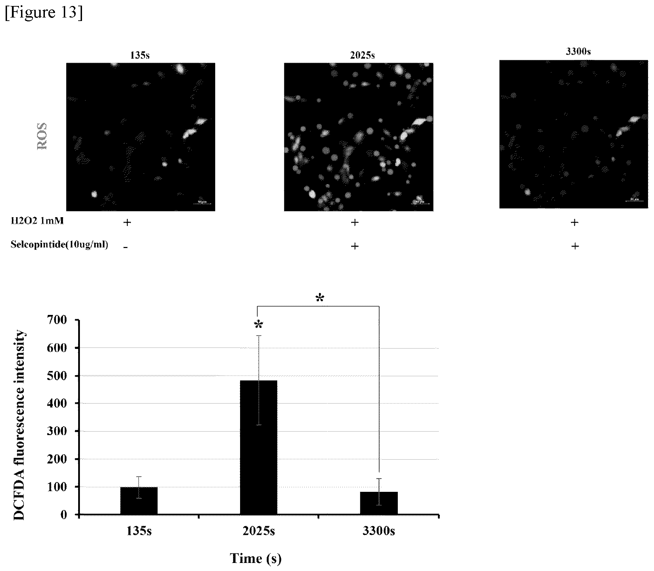

Referring to FIG. 13, it was confirmed that after treatment with 1 mM H₂O₂, the amount of reactive oxygen species reached its maximum at approximately 30 minutes, then decreased and became significant at approximately one hour. Based on these results, it was confirmed that Selcopintide has the potential to scavenge reactive oxygen species.

### Example 15: Confirmation of mRNA levels of endogenous antioxidant enzymes in hDPC cells by Selcopintide using RT-PCR

Cultured hDPCs were seeded into 60 mm culture dishes at a density of 4 x 10⁵ cells, cultured for 24 hours, and then treated with 1 mM H₂O₂ and treated with the CPNE7 recombinant protein (100 ng/mL) and Selcopintide (10 ug/mL). After 30 minutes and one hour, the mRNA levels of Sod1, 2, and 3, catalase, Gpx7, Hmox1, and Txrnd1 were checked.

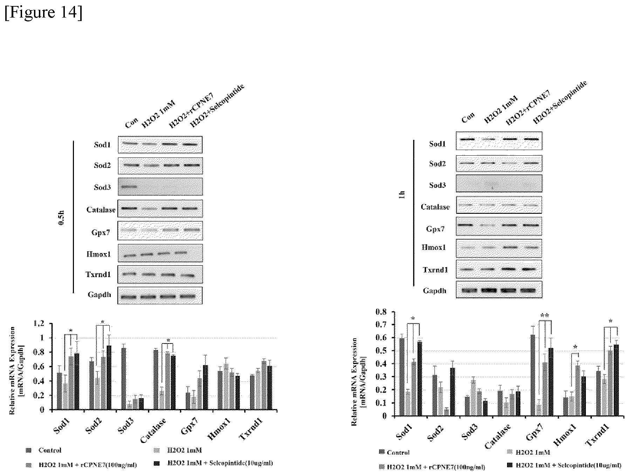

Referring to FIG. 14, it can be seen that the endogenous antioxidant enzymes Sod1, 2, and catalase significantly increased after 30 minutes of treatment with the CPNE7 recombinant protein and Selcopintide compared to the H₂O₂-only-treated group, and the endogenous antioxidant enzymes Sod1 and Gpx7, and Hmox1 and Txrnd1, which inhibit the formation of OH free radicals, significantly increased after one hour of treatment with the CPNE7 recombinant protein and Selcopintide.

Based on the above results, it was confirmed that Selcopintide can scavenge reactive oxygen species by controlling the endogenous antioxidant enzymes similar to the CPNE7 protein.

### Example 16: Comparative Confirmation of degree of DNA damage through immunofluorescence staining with γ-H2A.X which is a DNA damage marker protein

As described above, it was confirmed that the CPNE7 recombinant protein suppresses cell aging by scavenging excessive reactive oxygen species in hDPCs to prevent or repair DNA damage.

Cultured hDPCs were seeded into 35 mm culture dishes for a confocal microscope at a density of 1 x 10⁵ cells, cultured for 24 hours, and then treated with 1 mM H₂O₂ and treated with 10 ug/mL of Selcopintide. After 24 hours, the cells were fixed with 4% paraformaldehyde.

Immunofluorescence staining using a γ-H2A.X antibody was performed on the fixed cells to compare intensity.

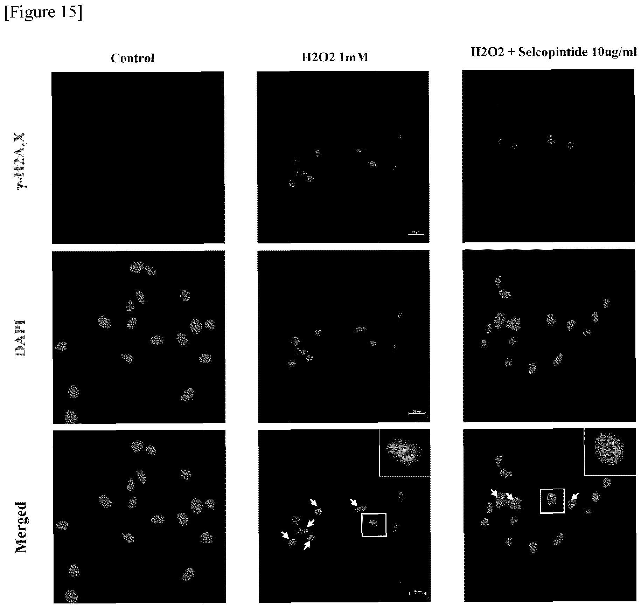

Referring to FIG. 15, it was confirmed that a significant amount of DNA damage was induced in the group treated with 1 mM H₂O₂ to induce oxidative stress, whereas the accumulation of DNA damage was significantly reduced in the group treated with 10 ug/mL of Selcopintide.

Based on the above results, it was confirmed that Selcopintide prevents or repairs DNA damage by scavenging excessive reactive oxygen species similarly to the CPNE7 protein.

### Example 17-1: Measurement of amount of living cells using highly sensitive water-soluble tetrazolium salt (WST-8)

In the study on odontoblasts examined above, it was confirmed that the CPNE7 protein and Selcopintide have the ability to scavenge reactive oxygen species. Next, the peptide's ability to scavenge reactive oxygen species was also examined in cardiomyocytes, which are known to differentiate from mesenchymal stem cells (MSCs), similar to odontoblasts.

Cultured cardiomyocytes (H9C2 cell line) were seeded into 96-well plates at a density of 3 x 10³ cells per well, cultured for 24 hours, and then treated with hydrogen peroxide (H₂O₂) at concentrations of 0u M (Control), 50 uM, 100 uM, 200 uM, 500 uM, 1 mM, and 2 mM for 24 hours. The WST-8 solution was added to each well and allowed to react for one hour, and then absorbance was measured at 450 nm using a microplate reader and compared.

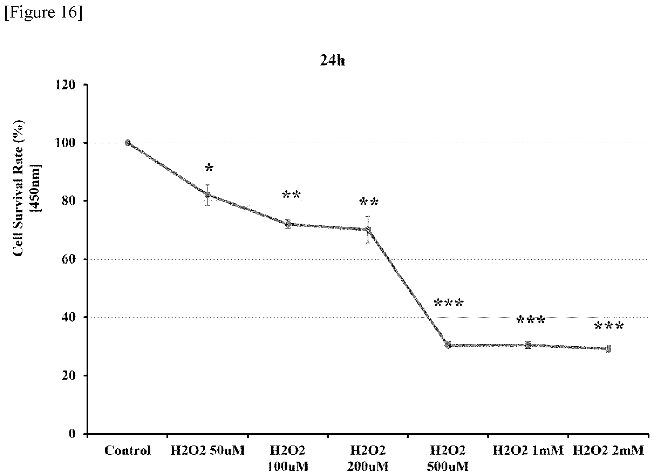

As shown in FIG. 16, it was confirmed that cell viability decreased as the concentration of H₂O₂ increased, and similar cell viability was observed at a H₂O₂ concentration of 500 uM or higher.

Thereafter, the concentration of 1 mM H₂O₂ was selected as the concentration that induces oxidative stress in H9C2 cells.

### Example 17-2: Confirmation of cells under optical microscope and measurement of amount of living cells using highly sensitive water-soluble tetrazolium salt (WST-8) after treating cells with H₂O₂, which induces intracellular reactive oxygen species, and treating cells with Selcopintide

Cultured H9C2 cells were seeded into 6-well plates at a density of 1 x 10⁵ cells per well, cultured for 24 hours, and then treated with 1 mM hydrogen peroxide (H₂O₂) and treated with Selcopintide at concentrations of 1 ug/mL, 10 ug/mL, 100 ug/mL, and 500 ug/mL for 24 hours. Untreated H9C2 cells were used as the control.

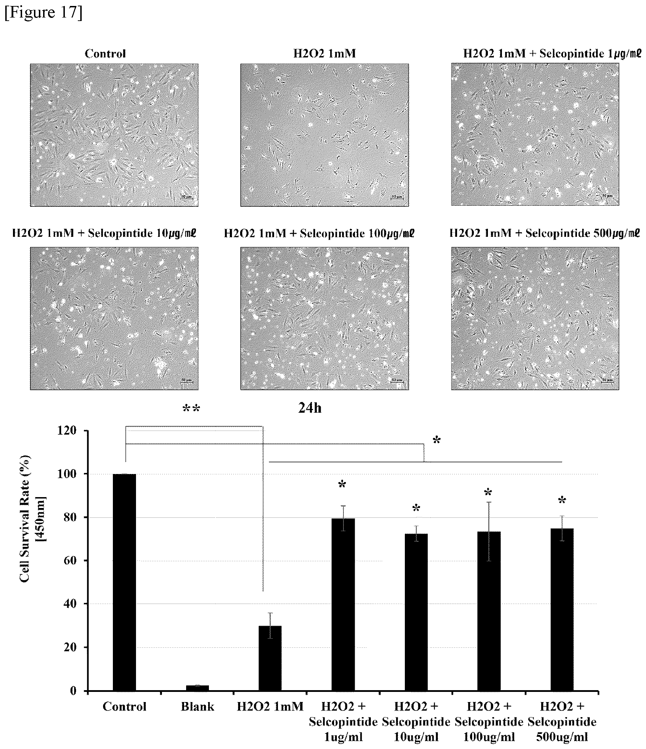

In the 1 mM H₂O₂-treated group, significant apoptosis of cardiomyocytes was observed compared to the control. On the other hand, it was confirmed that cell viability was higher in the Selcopintide-treated groups compared to the H₂O₂-only-treated group (FIG. 17).

Cultured H9C2 cells were seeded into 96-well plates at a density of 3 x 10³ cells per well, cultured for 24 hours, and then treated with 1 mM hydrogen peroxide (H₂O₂) and treated with Selcopintide at concentrations of 1 ug/mL, 10 ug/mL, 100 ug/mL, and 500 ug/mL for 24 hours. Untreated H9C2 cells were used as the control.

The WST-8 solution was added to each well and allowed to react for 1 hour, and then absorbance was measured at 450 nm using a microplate reader and compared.

It was confirmed that cell viability significantly decreased in the H₂O₂-only-treated group compared to the control, but cell viability was significantly higher in the 1 ug/mL, 10 ug/mL, 100 ug/mL, and 500 ug/mL Selcopintide-treated groups compared to the H₂O₂-only-treated group (FIG. 17).

Based on the above results, it was confirmed that the CPNE7 protein-derived functional peptide (Selcopintide) also has the ability to scavenge reactive oxygen species in cardiomyocytes.

### Example 18: Comparison of degree of cell aging through staining with beta-galactosidase (B-gal) which is a cell aging marker enzyme

In FIG. 17, it was confirmed that Selcopintide is effective in scavenging excessive reactive oxygen species in H9C2 cells, and its effect on cell aging was investigated.

Cultured H9C2 was seeded into 6-well plates at a density of 1 x 10⁵ cells, cultured for 24 hours, and then treated with 1 mM H₂O₂ and treated with 1 ug/mL, 10 ug/mL, 100 ug/mL, and 500 ug/mL of Selcopintide. After 24 hours, the cells were fixed with 4% paraformaldehyde.

Next, B-gal staining was performed on the fixed cells for 24 hours to compare the degree of cell aging.

24 hours after treatment with 1 mM H₂O₂, a large number of B-gal positive H9C2 cells were identified. On the other hand, it was confirmed that the number of B-gal positive H9C2 cells was significantly reduced in the Selcopintide-treated groups, compared to the H₂O₂-only-treated group.

Based on the above results, it was analyzed that the reactive oxygen species scavenging ability of Selcopintide can inhibit cardiomyocyte aging.

### Example 19: Measurement of amount of living cells using highly sensitive water-soluble tetrazolium salt (WST-8)

In previous studies on odontoblasts and cardiomyocytes, it was confirmed that Selcopintide has the ability to scavenge reactive oxygen species. Next, the present inventors attempted to determine whether the peptide also has the ability to scavenge reactive oxygen species in dermal fibroblasts, which are known to differentiate from mesenchymal stem cells (MSCs), similar to odontoblasts and cardiomyocytes.

Cultured dermal fibroblast (CCD986SK) cells were seeded into 96-well plates at a density of 3 x 10³ cells per well, cultured for 24 hours, and then treated with hydrogen peroxide (H₂O₂) at concentrations of 0 uM (Control), 50 uM, 100 uM, 200 uM, 500 uM, 1 mM, and 2 mM for 24 hours. The WST-8 solution was added to each well and allowed to react for one hour, and then absorbance was measured at 450 nm using a microplate reader and compared.

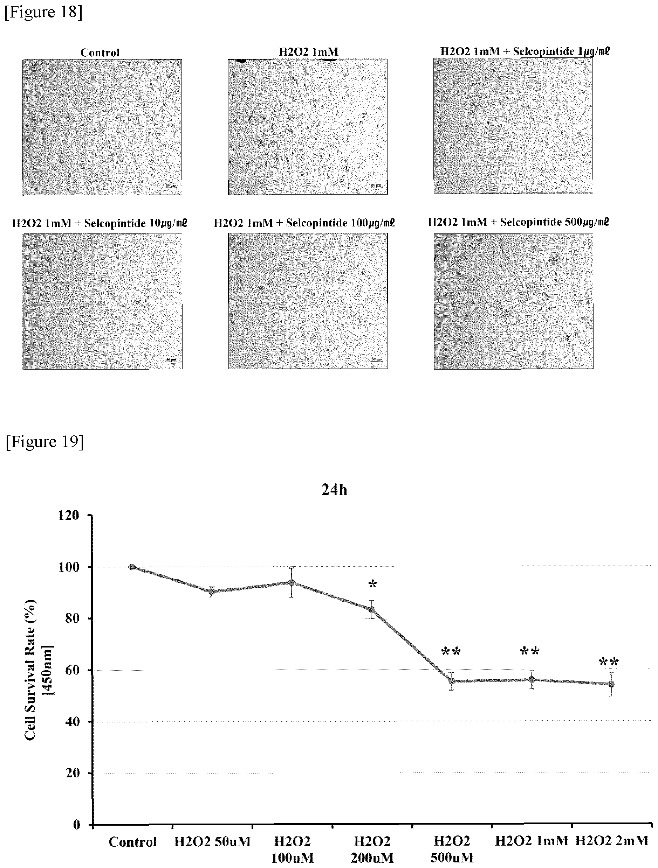

As shown in FIG. 19, it was confirmed that cell viability decreased as the concentration of H₂O₂ increased, and similar cell viability was observed at a H₂O₂ concentration of 500 uM or higher.

Thereafter, the concentration of 1 mM H₂O₂ was selected as the concentration that induces oxidative stress in CCD986SK cells.

### Example 20: Confirmation of cells under optical microscope and measurement of amount of living cells using highly sensitive water-soluble tetrazolium salt (WST-8) after treating cells with H₂O₂, which induces intracellular reactive oxygen species, and treating cells with Selcopintide

Cultured CCD986SK cells were seeded into 6-well plates at a density of 1 x 10⁵ cells per well, cultured for 24 hours, and then treated with 1 mM hydrogen peroxide (H₂O₂) and treated with Selcopintide at concentrations of 1 ug/mL, 10 ug/mL, 100 ug/mL, and 500 ug/mL for 24 hours. Untreated CCD986SK cells were used as the control.

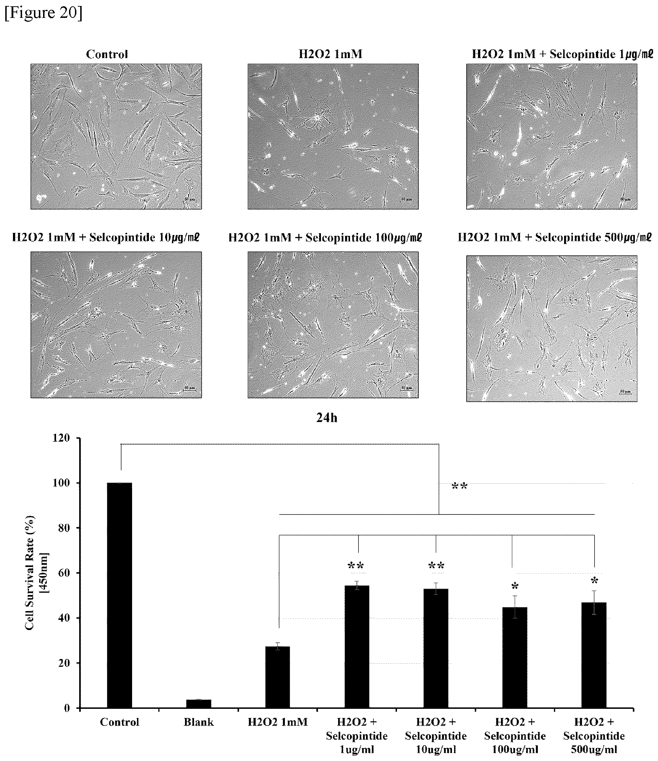

In the 1 mM H₂O₂-treated group, significant apoptosis of dermal fibroblasts was observed compared to the control. On the other hand, it was confirmed that cell viability was higher in the Selcopintide-treated groups, compared to the H₂O₂-only-treated group. In particular, the significance was highest at 1 ug/mL and 10 ug/mL of Selcopintide (FIG. 20).

Cultured CCD986SK cells were seeded into 96-well plates at a density of 3 x 10³ cells per well, cultured for 24 hours, and then treated with 1 mM hydrogen peroxide (H₂O₂) and treated with Selcopintide at concentrations of 1 ug/mL, 10 ug/mL, 100 ug/mL, and 500 ug/mL for 24 hours. Untreated CCD986SK cells were used as the control.

The WST-8 solution was added to each well and allowed to react for one hour, and then absorbance was measured at 450 nm using a microplate reader and compared.

It was confirmed that cell viability significantly decreased in the H₂O₂-only-treated group compared to the control, but cell viability was significantly higher in the 1 ug/mL, 10 ug/mL, 100 ug/mL, and 500 ug/mL Selcopintide-treated groups, compared to the H₂O₂-only-treated group (FIG. 20).

As a result, it was confirmed that the CPNE7 protein-derived functional peptide (Selcopintide) also has the ability to scavenge reactive oxygen species in dermal fibroblasts.

### Example 21: Comparison of degree of cell aging through staining with beta-galactosidase (B-gal) which is a cell aging marker enzyme

As shown in FIG. 20, it was confirmed that Selcopintide is effective in scavenging excessive reactive oxygen species in CCD986SK cells, and its effect on cell aging was investigated.

Cultured CCD986SK cells were seeded into 6-well plates at a density of 1 x 10⁵ cells, cultured for 24 hours, and then treated with 1 mM H₂O₂ and treated with 1 ug/mL, 10 ug/mL, 100 ug/mL, and 500 ug/mL of Selcopintide. After 24 hours, the cells were fixed with 4% paraformaldehyde.

B-gal staining was performed on the fixed cells for 24 hours to compare the degrees of cell aging.

24 hours after treatment with 1 mM H₂O₂, a large number of B-gal positive CCD986SK cells were identified. On the other hand, it was confirmed that the number of B-gal positive CCD986SK cells was significantly reduced in the Selcopintide-treated groups, compared to the H₂O₂-only-treated group.

Based on the above results, it was analyzed that the reactive oxygen species scavenging ability of Selcopintide can inhibit the aging of dermal fibroblasts.

Through the examples as described above, it can be seen that the CPNE7 protein and the CPNE7 protein-derived functional peptide (Selcopintide) can be used to prevent or treat dental pulp diseases, myocardial infarction, skin inflammation, and the like caused by excessive reactive oxygen species (H₂O₂), thereby preventing the aging of a subject.

The present specification and drawings have disclosed preferred embodiments of the present invention, and although specific terms have been used, they are used only in a general sense to easily explain the technical contents of the present invention and to aid in understanding the invention, and are not intended to limit the scope of the present invention. It will be obvious to those skilled in the art that other changes and modifications based on the technical spirit of the present invention can be implemented in addition to the embodiments disclosed herein.

## Claims

1. A composition for scavenging intracellular reactive oxygen species, comprising a CPNE7 protein or a peptide consisting of any one amino acid sequence of SEQ ID NOs: 1 to 96.

2. The composition of claim 1, wherein the cells are dental pulp cells, cardiomyocytes, or dermal fibroblasts.

3. A peptide for scavenging intracellular reactive oxygen species, comprising any one amino acid sequence of SEQ ID NOs: 1 to 96.

4. A polynucleotide encoding the peptide of claim 3.

5. An expression vector comprising the polynucleotide of claim 4.

6. A pharmaceutical composition for preventing or treating a heart disease caused by oxidative stress in cardiomyocytes or a skin disease caused by oxidative stress in dermal fibroblasts, comprising the peptide of claim 3.

7. The pharmaceutical composition of claim 6, wherein the pharmaceutical composition includes a polypeptide in which the peptide is repeatedly linked.

8. The pharmaceutical composition of claim 6, wherein the pharmaceutical composition includes a complex in which a drug, which exhibits a preventive or therapeutic effect on a heart disease caused by oxidative stress in cardiomyocytes or a skin disease caused by oxidative stress in dermal fibroblasts, is combined with the peptide.

9. The pharmaceutical composition of claim 6, wherein the pharmaceutical composition further comprises a pharmaceutically acceptable carrier, excipient, or diluent.

10. The pharmaceutical composition of claim 6, wherein the heart disease is coronary artery disease (CAD), myocardial infarction (MI), heart failure, or atrial fibrillation (AF).

11. The pharmaceutical composition of claim 6, wherein the skin disease is photodermatitis or atopic dermatitis.

12. A quasi-drug composition for preventing or ameliorating a heart disease caused by oxidative stress in cardiomyocytes or a skin disease caused by oxidative stress in dermal fibroblasts, comprising the peptide of claim 3.

13. The quasi-drug composition of claim 12, wherein the quasi-drug composition is provided in the form of an ointment, a patch, a powder, a gel, a tablet, or a spray.

14. A health functional food composition for preventing or ameliorating a heart disease caused by oxidative stress in cardiomyocytes or a skin disease caused by oxidative stress in dermal fibroblasts, comprising the peptide of claim 3.

15. The health functional food composition of claim 14, wherein the health functional food composition is used in the manufacture of beverages, teas, spices, gums, or confectioneries.
